(19) Europäisches Patentamt
European Patent Office
Office européen des brevets

(11) Publication number: **0 666 253 A1**

# (12) EUROPEAN PATENT APPLICATION published in accordance with Art. 158(3) EPC

(21) Application number: **93923036.3**

(22) Date of filing: **20.10.93**

(86) International application number: **PCT/JP93/01516**

(87) International publication number: **WO 94/08956 (28.04.94 94/10)**

(51) Int. Cl.[6]: **C07C 317/36**, C07C 317/40, C07C 323/37, C07C 323/41, A61K 31/135, A61K 31/14, A61K 31/16

(30) Priority: **21.10.92 JP 283225/92**

(43) Date of publication of application: **09.08.95 Bulletin 95/32**

(84) Designated Contracting States: **AT BE CH DE DK ES FR GB GR IE IT LI LU MC NL PT SE**

(71) Applicant: **TAISHO PHARMACEUTICAL CO. LTD**
**24-1 Takata 3-chome**
**Toshima-ku**
**Tokyo 171 (JP)**
Applicant: **MITSUI PETROCHEMICAL INDUSTRIES, LTD.**
**2-5, Kasumigaseki 3-chome**
**Chiyoda-ku**
**Tokyo 100 (JP)**

(72) Inventor: **MIYOSHI, Toshio, Taisho Pharmaceutical Co., Ltd.**
**24-1, Takata 3-chome,**
**Toshima-ku**
**Tokyo 171 (JP)**
Inventor: **ASAMI, Yumiko, Taisho Pharmaceutical Co., Ltd.**
**24-1, Takata 3-chome,**
**Toshima-ku**
**Tokyo 171 (JP)**
Inventor: **SATO, Masakazu, Taisho Pharmaceutical Co., Ltd.**
**24-1, Takata 3-chome,**
**Toshima-ku**
**Tokyo 171 (JP)**
Inventor: **ISHIGURO, Masaharu, Mitsui Petrochemical Ind., Ltd**
**1-2, Waki 6-chome,**
**Waki-cho**
**Kuga-gun,**
**Yamahi-ken 740 (JP)**
Inventor: **KITAHARA, Takumi, Mitsui Petrochemical Ind., Ltd.**
**1-2, Waki 6-chome,**
**Waki-cho**
**Kuga-gun,**
**Yamaguchi-ken 740 (JP)**
Inventor: **TOMINO, Ikuo, Mitsui Petrochemical Ind., Ltd.**
**1-2, Waki 6-chome,**
**Waki-cho**
**Kuga-gun,**
**Yamaguchi-ken 740 (JP)**

(74) Representative: **Cresswell, Thomas Anthony**
**J.A. KEMP & CO.**
**14 South Square**
**Gray's Inn**
**London WC1R 5LX (GB)**

## (54) N-t-BUTYLANILINE ANALOG AND LIPID LEVEL DEPRESSANT CONTAINING THE SAME.

(57) An N-t-butylaniline analog represented by general formula (I) or a salt thereof, and a lipid level depressant containing the same as the active ingredient. In said formula, $R^1$ and $R^2$, which may be the same or different from each other, represent each hydrogen, halogen, amino, nitro, acetamide, lower alkyl, lower alkoxy,



Rank Xerox (UK) Business Services
(3. 10/3.09/3.3.4)

halogenated lower alkyl, halogenated lower alkoxy or lower alkanoyl; $R^3$ represents hydrogen, halogen, lower alkoxy or halogenated lower alkyl; and Z represents S, SO or $SO_2$. This compound has a remarkably excellent effect of depressing the lipid level as compared with clofibrate and 4-(N-t-butyl)methanesulfonylaminodiphenyl ether and is extremely reduced in toxicity.

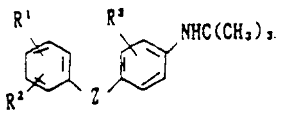

(I)

## TECHNICAL FIELD

The present invention relates to novel N-t-butylaniline analogs and the use of the same as lipid level depressant.

## BACKGROUND ART

Arteriosclerosis, which has become a problem as one of adult diseases at present, is known to be closely related to the high-lipid blood resulting from the increase of lipids in blood, such as cholesterol, triglyceride, etc. In recent years, the study on lipid has been made at the level of lipoprotein, and it was revealed that arteriosclerosis can be effectively prevented and/or cured by an increase in high density lipoprotein (HDL). Although clofibrate has been widely used as lipid level depressant, this depressant is not satisfactory owing to side effects such as hepatic malfunction, anorexia, muscular pain, etc.

Some of the compounds of the invention fall under the general formula representing the photoconductive compounds disclosed in GB Patent No. 1294136, but there is not any specific description or suggestion of the compounds of the invention. Although compounds similar in structure to the compounds of the invention are disclosed in Japanese Patent Laid-Open Publication (Kokai) No. 126155/1987, the action of these compounds on the depression of the lipid level in blood is not reported. U.S. Patents Nos. 3531523 and 3553329 disclose N-(phenoxyphenyl)sulfamide derivatives, but the action of these compounds on the depression of the lipid level in blood was not strong.

## DISCLOSURE OF INVENTION

The object of the present invention is to provide a pharmaceutical preparation functioning as lipid level depressant in blood, by which cholesterol and triglyceride are decreased in blood simultaneously with an increase in HDL with less side effects.

The present inventors have, as a result of their eager research, found novel N-t-butylaniline analogs depressing the lipid level in blood.

The present invention relates to N-t-butylaniline analog represented by formula (I):

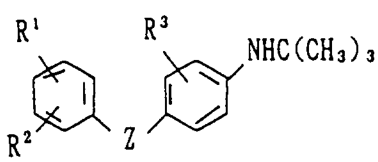

(I)

wherein $R^1$ and $R^2$ are the same or different and represent each a hydrogen atom, halogen atom, amino group, nitro group, acetamido group, lower alkyl group, lower alkoxy group, halogeno lower alkyl group, halogeno lower alkoxy group or lower alkanoyl group, $R^3$ represents a hydrogen atom, halogen atom, lower alkoxy group or halogeno lower alkyl group, and Z represents S, SO or $SO_2$, as well as a salt thereof. The present invention further relates to a lipid level depressant containing the same.

In the invention, the halogen atom includes a fluorine atom, chlorine atom, bromine atom and iodine atom. The lower alkyl group includes a $C_1$-$C_4$ straight-chain or branched alkyl group, such as a methyl group, ethyl group, propyl group, isopropyl group, butyl group, isobutyl group, s-butyl group and t-butyl group. The lower alkoxy group includes a $C_1$-$C_4$ straight-chain or branched alkoxy group, such as methoxy group, ethoxy group, propoxy group, isopropoxy group, butoxy group, isobutoxy group, s-butoxy group and t-butoxy group. The halogeno lower alkyl group includes a $C_1$-$C_3$ straight-chain or branched alkyl group substituted with halogen atom(s), such as chloromethyl group, dichloroethyl group, trifluoromethyl group, etc. The halogeno lower alkoxy group includes a $C_1$-$C_3$ straight-chain or branched alkoxy group substituted with halogen atom(s), such as chloromethoxy group, dichloroethoxy group, trifluoromethoxy group, etc. The lower alkanoyl group includes a $C_1$-$C_4$ straight-chain or branched alkanoyl group, such as acetyl group, propionyl group and butyryl group.

Examples of compounds of formula (I) wherein Z is S, include:

(1) 4-(t-butylamino)diphenylsulfide
(2) N-t-butyl-4-(4-isopropylphenylthio)aniline
(3) N-t-butyl-4-(4-tolylthio)aniline
(4) N-t-butyl-4-(4-methoxyphenylthio)aniline

(5) N-t-butyl-4-(4-bromophenylthio)aniline
(6) N-t-butyl-4-(3-methoxyphenylthio)aniline
(7) N-t-butyl-4-(3-trifluoromethylphenylthio)aniline
(8) N-t-butyl-4-(3-chlorophenylthio)aniline
(9) N-t-butyl-4-(2-chlorophenylthio)aniline
(10) N-t-butyl-4-(2,5-dichlorophenylthio)aniline
(11) N-t-butyl-3-chloro-4-(phenylthio)aniline
(12) N-t-butyl-4-(4-aminophenylthio)aniline
(13) N-t-butyl-4-(4-nitrophenylthio)aniline
(14) N-t-butyl-4-(4-acetamidophenylthio)aniline
(15) N-t-butyl-4-(4-trifluoromethoxyphenylthio)aniline
(16) N-t-butyl-4-(4-acetylphenylthio)aniline
(17) N-t-butyl-2-methoxy-4-(phenylthio)aniline
(18) N-t-butyl-3-trifluoromethyl-4-(phenylthio)aniline

Examples of compounds of formula (I) wherein Z is SO, include:

(1) 4-(t-butylamino)diphenylsulfoxide
(2) N-t-butyl-4-(4-isopropylphenylsulfinyl)aniline
(3) N-t-butyl-4-(4-tolylsulfinyl)aniline
(4) N-t-butyl-4-(4-methoxyphenylsulfinyl)aniline
(5) N-t-butyl-4-(4-bromophenylsulfinyl)aniline
(6) N-t-butyl-4-(3-methoxyphenylsulfinyl)aniline
(7) N-t-butyl-4-(3-trifluoromethylphenylsulfinyl)aniline
(8) N-t-butyl-4-(3-chlorophenylsulfinyl)aniline
(9) N-t-butyl-4-(2-chlorophenylsulfinyl)aniline
(10) N-t-butyl-4-(2,5-dichlorophenylsulfinyl)aniline
(11) N-t-butyl-3-chloro-4-(phenylsulfinyl)aniline
(12) N-t-butyl-4-(4-aminophenylsulfinyl)aniline
(13) N-t-butyl-4-(4-nitrophenylsulfinyl)aniline
(14) N-t-butyl-4-(4-acetamidophenylsulfinyl)aniline
(15) N-t-butyl-4-(4-trifluoromethoxyphenylsulfinyl)aniline
(16) N-t-butyl-4-(4-acetylphenylsulfinyl)aniline
(17) N-t-butyl-2-methoxy-4-(phenylsulfinyl)aniline
(18) N-t-butyl-3-trifluoromethyl-4-(phenylsulfinyl)aniline

Examples of compounds of formula (I) wherein Z is $SO_2$ , include:

(1) 4-(t-butylamino)diphenylsulfone
(2) N-t-butyl-4-(4-isopropylphenylsulfonyl)aniline
(3) N-t-butyl-4-(4-tolylsulfonyl)aniline
(4) N-t-butyl-4-(4-methoxyphenylsulfonyl)aniline
(5) N-t-butyl-4-(4-bromophenylsulfonyl)aniline
(6) N-t-butyl-4-(3-methoxyphenylsulfonyl)aniline
(7) N-t-butyl-4-(3-trifluoromethylphenylsulfonyl)aniline
(8) N-t-butyl-4-(3-chlorophenylsulfonyl)aniline
(9) N-t-butyl-4-(2-chlorophenylsulfonyl)aniline
(10) N-t-butyl-4-(2,5-dichlorophenylsulfonyl)aniline
(11) N-t-butyl-3-chloro-4-(phenylsulfonyl)aniline
(12) N-t-butyl-4-(4-aminophenylsulfonyl)aniline
(13) N-t-butyl-4-(4-nitrophenylsulfonyl)aniline
(14) N-t-butyl-4-(4-acetamidophenylsulfonyl)aniline
(15) N-t-butyl-4-(4-trifluoromethoxyphenylsulfonyl)aniline
(16) N-t-butyl-4-(4-acetylphenylsulfonyl)aniline
(17) N-t-butyl-2-methoxy-4-(phenylsulfonyl)aniline
(18) N-t-butyl-3-trifluoromethyl-4-(phenylsulfonyl)aniline

The salt refers to a pharmaceutically acceptable salt to which an inorganic or organic acid has been added. Examples are hydrochloride, hydrobromide, hydroiodide, sulfate, nitrate, phosphate, acetate, fumarate, maleate, methanesulfonate, etc.

Those compounds of formula (I) wherein Z is S (referred to hereinafter as Compound (I')) can be produced by reacting Compound (II) with t-butanol in the presence of an acid as follows:

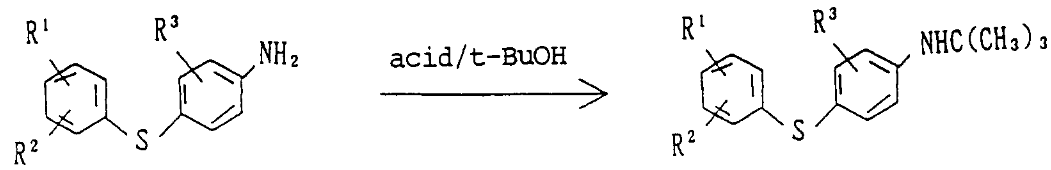


(II) (I')

wherein $R^1$, $R^2$ and $R^3$ possess the same meanings as defined above.

The acid used in the above reaction includes a protonic acid such as hydrochloric acid, sulfuric acid, phosphoric acid, acetic acid, 4-toluenesulfonic acid, etc., Lewis acid such as boron trifluoride ether complex, aluminum chloride, zinc chloride, etc., and a solid acid such as Amberlyst, Nafion, silica alumina, etc. t-Butanol is used preferably in a molar ratio of from 1 to 30, more preferably 3 to 15, relative to Compound (II). The reaction is conducted usually in an autoclave at about 100-170 °C.

In the presence of an oxidizing agent such as m-chloroperbenzoic acid, hydrogen peroxide, etc., Compound (I') can be converted in a usual manner into those compounds of formula (I) wherein Z is SO or $SO_2$.

The resulting reaction mixture containing Compound (I) is extracted and concentrated, followed by isolation of Compound (I) or a salt thereof by separation means known to the art, such as recrystallization after conversion into a salt, distillation of the free form, chromatographic separation, etc. Compound (II) used as the starting material can be obtained according to the following known method:

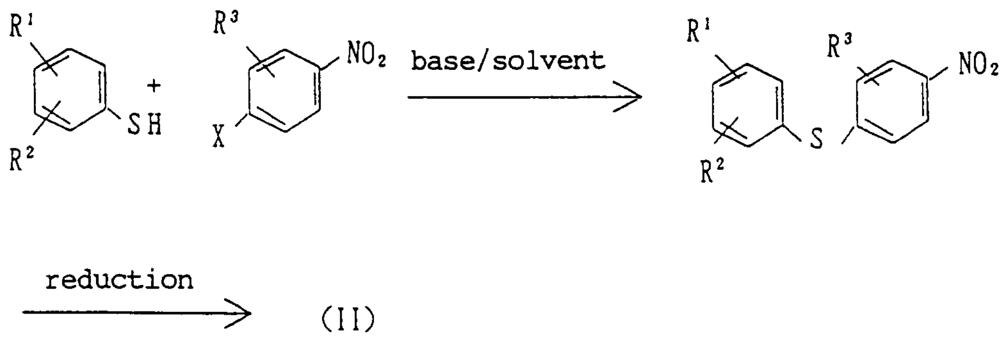


(II)

wherein X represents a halogen atom; and $R^1$, $R^2$ and $R^3$ possess the same meanings as defined above.

Compound (I) can be converted in a usual manner into the above-mentioned pharmaceutically acceptable acid-addition salt.

For use as lipid level depressant, the compound of the invention is administered orally or parenterally in the form of tablets, pills, capsules, granules, oil suspension, syrup, etc. According to the conventional pharmaceutical manufacturing, each form of preparation is produced if necessary with the addition of generally used filler, such as calcium carbonate, calcium phosphate, cellulose, starch, glucose, lactose, carboxymethylcellulose calcium, talc, magnesium stearate, etc. Since the compound of the invention turns water-soluble when converted into an acid-addition salt, an injection can also be prepared using the salt.

The compound of the invention can be administered orally or parenterally to an adult patient at intervals in a dosage of from 10 to 3000 mg/day. This dosage may be suitably varied depending on the type of disease as well as the age, body weight and symptoms of the patient.

BEST MODE FOR CARRYING OUT THE INVENTION

The invention is described in more detail with reference to the following examples and test examples.

(Reference Example 1) 4-nitrodiphenylsulfide

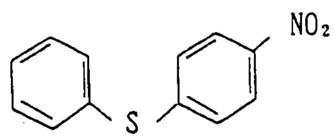

110 g (1.0 mol) of thiophenol, 174 g (1.1 mol) of 4-chloronitrobenzene and 152 g (1.1 mol) of potassium carbonate were mixed in 1 liter of N,N-dimethylformamide and then refluxed for 6 hours. The reaction mixture was cooled to room temperature, then the N,N-dimethylformamide was distilled off under reduced pressure, and the residue was extracted twice with 500 ml ethyl acetate. The resulting ethyl acetate extract was concentrated in an evaporator. 800 ml hexane was added to the concentrate, and the mixture was crystallized by stirring for 5 min. at room temperature. The crystals were filtered off to give 184 g of 4-nitrodiphenylsulfide as yellow crystals (80 % yield).

(Reference Example 2) 4-aminodiphenylsulfide hydrochloride

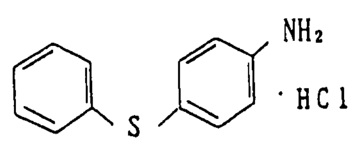

23.1 g (0.10 mol) of 4-nitrodiphenylsulfide and 16.8 g (0.30 mol) of reduced iron were suspended in 150 ml of 50 % ag. ethanol, then heated under reflux for 30 min. 1.1 ml of 10 % hydrochloric acid was added dropwise thereto for 15 min. under reflux, and the mixture was allowed to react for 5 hours. The reaction mixture was cooled to room temperature, and 300 ml ethyl acetate and 5 ml of 10 % aq. sodium hydroxide were added thereto. Subsequently, the reaction mixture was filtered through celite, and the organic layer from the filtrate was washed with 100 ml water. The resulting organic layer was concentrated in an evaporator, and the concentrate was dissolved in 130 ml methanol, and 9.03 g (0.09 mol) of conc. hydrochloric acid was added thereto, and the solution was concentrated again in an evaporator. The concentrate thus obtained was washed with 300 ml ethyl acetate and filtered with suction, and the filtrate was dried under reduced pressure to give 21.2 g of 4-aminodiphenylsulfide hydrochloride as pale green crystals (89 % yield).

(Reference Example 3) 4-(N-t-butyl)methanesulfonylaminodiphenyl ether

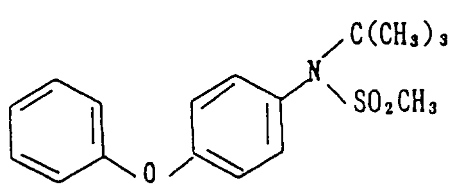

0.52 g of 4-(t-butylamino)diphenyl ether was dissolved in 1.1 ml pyridine, and 0.16 ml methanesulfonyl chloride was added thereto under cooling on ice. The solution was stirred for 20 min. under cooling on ice and then the temperature was raised to room temperature. Addition of 30 ml water caused precipitation of

crystals. These crystals were then filtered off and dried under reduced pressure to give 0.23 g of 4-(N-t-butyl)methanesulfonylaminodiphenyl ether as white crystals (34 % yield).

(Reference Example 4) N,N-diethyl-4-(4-tolylthio)aniline

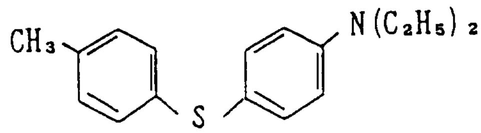

1.72 g (0.008 mol) of 4-(4-tolylthio)aniline, 1.74 g (0.016 mol) of ethyl bromide and 1.05 g (0.016 mol) of 85 % potassium hydroxide were dissolved in 40 ml acetone and then allowed to react at 60 °C for 8 hours. The reaction mixture was concentrated under reduced pressure and extracted with 50 ml ethyl acetate and 20 ml water. The organic layer was dried over anhydrous magnesium sulfate and concentrated under reduced pressure. The concentrate was separated by column chromatography on silica gel with hexane/ethyl acetate (V/V = 9/1) as an eluent, to give 0.8 g of N,N-diethyl-4-(4-tolylthio)aniline as oil (37 % yield).
n.m.r. ($\delta$ value, $CDCl_3$): 1.17 (6H, t, J = 7 Hz), 2.27 (3H, s), 3.36 (4H, q, J = 7 Hz), 6.5-7.4 (8H, m).

(Example 1) 4-(t-butylamino)diphenylsulfide hydrochloride

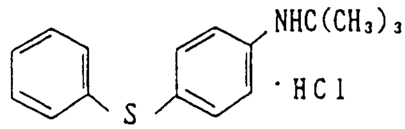

2.38 g (0.010 mol) of 4-aminodiphenylsulfide hydrochloride and 7.40 g (0.10 mol) of t-butanol were introduced to a 50 ml glass-lined SUS316 autoclave and allowed to react at 130 °C for 8 hours. The reaction solution was cooled to room temperature and extracted with 30 ml ethyl acetate and 30 ml of 2 % aq. sodium hydroxide, and the organic layer was washed with 20 ml water. The organic layer was concentrated in an evaporator, and the concentrate was subjected to silica gel column chromatography with hexane/ethyl acetate = 5/1 (v/v) as an eluent, to give 1.31 g of 4-(t-butylamino)diphenylsulfide as pale brown crystals (51 % yield). The 4-(t-butylamino)diphenylsulfide thus obtained was dissolved in 10 ml methanol, followed by addition of 0.51 g of conc. hydrochloric acid. Then, the solution was concentrated in an evaporator, and the concentrate was dissolved again in 30 ml methanol, then 0.3 g activated carbon was added thereto, and the solution was stirred for 2 min. at room temperature. The activated carbon was filtered off through celite, and the filtrate was concentrated. The concentrate was washed with 10 ml ethyl acetate and dried under reduced pressure to give 1.42 g of 4-(t-butylamino)diphenylsulfide hydrochloride (test compound No. 1) as white crystals (95 % yield from the free form). The physical properties of 4-(t-butylamino)diphenylsulfide and its hydrochloride are as follows:
4-(t-butylamino)diphenylsulfide
melting point: 44-45 °C.
n.m.r. ($\delta$ value, $CDCl_3$): 1.38 (9H, s), 3.46 (1H, br, s), 6.68 (2H, d, J = 8 Hz), 7.14 (5H, m), 7.28 (2H, d, J = 8 Hz).
4-(t-butylamino)diphenylsulfide hydrochloride
melting point: 176-177 °C.
n.m.r. ($\delta$ value, $CDCl_3$): 1.40 (9H, s), 7.16 (2H, d, J = 8 Hz), 7.3-7.6 (7H, m).

(Examples 2 to 18) 4-(t-butylamino)diphenylsulfide analogs

The same procedure as in Example 1 was followed using each of substituted 4-aminodiphenylsulfide hydrochlorides in place of 4-aminodiphenylsulfide hydrochloride. The results are shown in Table 1. The physical properties of these substituted 4-(t-butylamino) diphenylsulfide derivatives and the hydrochlorides thereof are shown in Tables 2 and 3, respectively.

Table 1

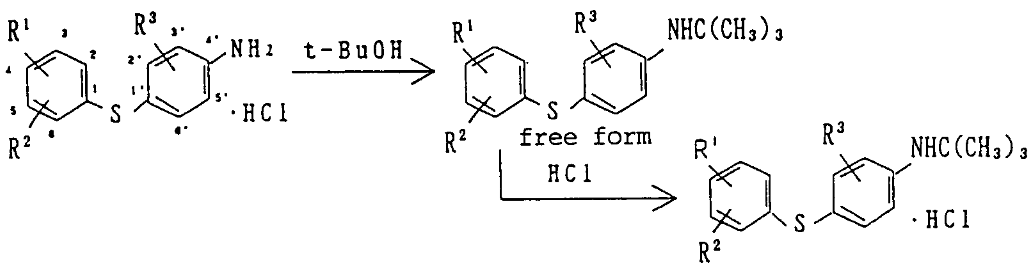


| Example | $R^1$, $R^2$ | $R^3$ | yield of t-butyl amino derivative (% based on amino derivative hydrochloride) | yield of t-butyl amino derivative hydrocloride (% from free form) |
|---|---|---|---|---|
| 2 | 4-iPr | H | 44 | 78 |
| 3 | 4-$CH_3$ | H | 54 | 77 |
| 4 | 4-$CH_3O$ | H | 33 | 99 |
| 5 | 4-Br | H | 17 | 98 |
| 6 | 3-$CH_3O$ | H | 48 | 96 |
| 7 | 3-$CF_3$ | H | 21 | 93 |
| 8 | 3-Cl | H | 54 | 79 |
| 9 | 2-Cl | H | 64 | 92 |
| 10 | 2,5-diCl | H | 57 | 84 |
| 11 | H | 2'-Cl | 59 | 89 |
| 12 | 4-$NH_2$ | H | 93 | 92 |
| 13 | 4-$NO_2$ | H | 35 | 95 |
| 14 | 4-$CH_3CONH$ | H | 75 | 90 |
| 15 | 4-$CF_3O$ | H | 35 | 97 |
| 16 | 4-$CH_3CO$ | H | 30 | 91 |
| 17 | H | 3'-$CH_3O$ | 23 | 90 |
| 18 | H | 2'-$CF_3$ | 42 | 95 |

Table 2 (1)

| $R^1$, $R^2$ | $R^3$ | m.p. (℃) | n. m. r. (δ value) ($CDCl_3$) |
|---|---|---|---|
| 4-iPr | H | 69~70 | 1.20 (6H, d, J=7Hz), 1.37 (9H, s), 2.84 (1H, m), 6.66 (2H, d, J=8Hz), 7.08 (4H, s), 7.26 (2H, d, J=8Hz) |
| 4-$CH_3$ | H | oil | 1.37 (9H, s), 2.2 (3H, s), 6.66 (2H, d, J=8Hz), 7.06 (4H, s, 7.26 (2H, d, J=8Hz) |
| 4-$CH_3O$ | H | oil | 1.37 (9H, s), 3.8 (3H, s), 6.67 (2H, d, J=9Hz), 6.82 (2H, d, J=9Hz), 7.22 (2H, d, J=9Hz), 7.25 (2H, d, J=9Hz), 2.7~3.4 (1H, br) |
| 4-Br | H | 106~109 | 1.37 (9H, s), 3.4~3.9 (1H, br), 6.66 (2H, d, J=9Hz), 6.94 (2H, d, J=9Hz), 7.25 (2H, d, J=9Hz), 7.28 (2H, d, J=9Hz) |

Table 2 (2)

| $R^1$, $R^2$ | $R^3$ | m.p. (℃) | n. m. r. (δ value) ($CDCl_3$) |
|---|---|---|---|
| 3-$CH_3O$ | H | oil | 1.38 (9H, s), 3.7 (3H, s), 6.6～7.4 (8H, m) |
| 3-$CF_3$ | H | oil | 1.44 (9H, s), 3.5 (br, 1H), 7.1～7.5 (3H, m), 6.70 (2H, d, J=7Hz), 7.30 (2H, d, J=7Hz), 7.25 (1H, s) |
| 3-Cl | H | 94～95 | 2.40 (9H, s), 6.6 (2H, d, J=9Hz), 7.04 (4H, m), 7.28 (2H, d, J=9Hz) |
| 2-Cl | H | 99～100 | 1.40 (9H, s), 3.4～4.4 (1H, br), 6.74 (4H, d, J=9Hz), 7.01 (2H, dd, J=3Hz, 3Hz), 7.31 (2H, d, J=9Hz) |
| 2,5-diCl | H | 79～80 | 1.41 (9H, s), 6.7 (2H, d, J=9Hz), 7.30 (2H, d, J=9Hz), 6.94 (1H, dd, J=3Hz, 9Hz), 6.50 (1H, dd, J=3Hz, 9Hz), 7.1～7.4 (1H, m), 2.8～4.0 (1H, br) |

Table 2 (5)

| $R^1$, $R^2$ | $R^3$ | m.p. (℃) | n. m. r. (δ value) ($CDCl_3$) |
|---|---|---|---|
| H | 2'-Cl | oil | 1.43 (9H, s), 2.4~3.4 (1H, br), 6.54 (1H, dd, J=3Hz, 9Hz), 6.83 (1H, d, J=3Hz), 7.0~7.4 (6H, m) |
| 4-$NH_2$ | H | oil | 1.31 (9H, s), 2.8~3.8 (3H, br), 6.54 (2H, d, J=9Hz), 6.62 (2H, d, J=9Hz), 7.10 (2H, d, J=9Hz), 7.14 (2H, d, J=9Hz) |
| 4-$NO_2$ | H | 115 ~117 | 1.43 (9H, s), 3.8~4.3 (1H, br), 6.73 (2H, d, J=9Hz), 7.09 (2H, d, J=9Hz), 7.30 (2H, d, J=9Hz), 8.02 (2H, d, J=9Hz) |
| 4-$CH_3CONH$ | H | oil | 1.36 (9H, s), 2.12 (3H, s), 3.0~3.5 (1H, br), 6.66 (2H, d, J=7Hz), 7.08 (2H, d, J=7Hz), 7.22 (2H, d, J=7Hz), 7.26 (2H, d, J=7Hz) |

Table 2 (a)

| $R^1$, $R^2$ | $R^3$ | m.p. (℃) | n. m. r. (δ value) ($CDCl_3$) |
|---|---|---|---|
| 4-$CF_3O$ | H | oil | 1.39 (9H, s), 6.67 (2H, d, J=9Hz), 7.07 (4H, m), 7.28 (2H, d, J=9Hz) |
| 4-$CH_3CO$ | H | oil | 1.38 (9H, s), 2.10 (3H, s), 3.8~4.3 (1H, br), 6.70 (2H, d, J=9Hz), 7.02 (2H, d, J=9Hz), 7.25 (2H, d, J=9Hz), 7.99 (2H, d, J=9Hz) |
| H | 3'-$CH_3O$ | oil | 1.42 (9H, s), 3.79 (3H, s), 4.0~5.0 (1H, br), 6.82 (1H, d, J=7Hz), 6.87 (1H, s), 7.0~7.4 (6H, m) |
| H | 2'-$CF_3$ | oil | 1.40 (9H, s), 3.8~4.2 (1H, br), 6.76 (1H, d, J=7Hz), 7.02 (1H, s), 7.31 (1H, d, J=7Hz), 7.1~7.3 (5H, m) |

Table 3 (i)

| $R^1$, $R^2$ | $R^3$ | m.p. (℃) | n.m.r. (δ value) ($CDCl_3$) |
|---|---|---|---|
| 4-iPr | H | 173~175 | 1.28 (6H, d, J=7Hz), 1.37 (9H, s), 2.94 (1H, m), 7.0~7.5 (8H, m) |
| 4-$CH_3$ | H | 177~179 | 1.38 (9H, s), 2.39 (3H, s), 7.08 (2H, d, J=8Hz), 7.28 (2H, d, J=8Hz), 7.40 (4H, m) |
| 4-$CH_3O$ | H | 79~81 | 1.38 (9H, s), 3.8 (3H, s), 7.10 (2H, d, J=9Hz), 7.19 (2H, d, J=9Hz), 7.57 (2H, d, J=9Hz), 7.62 (2H, d, J=9Hz), 11.2 (2H, br) |
| 4-Br | H | 106~109 | 1.40 (9H, s), 7.17 (2H, d, J=7Hz), 7.28 (2H, d, J=7Hz), 7.50 (4H, d, J=7Hz), 11.4 (2H, br) |

Table 3 (2)

| $R^1$, $R^2$ | $R^3$ | m.p. (℃) | n. m. r. (δ value) ($CDCl_3$) |
|---|---|---|---|
| 3-$CH_3O$ | H | 131 ～133 | 1.39 (9H, s), 3.80 (3H, s), 6.8～7.6 (8H, m), 11.16 (2H, br, s) |
| 3-$CF_3$ | H | 67 ～70 | 1.40 (9H, s), 7.4～7.8 (4H, m), 7.28 (2H, d, J=7Hz), 7.56 (2H, d, J=7Hz), 11.5 (2H, br) |
| 3-Cl | H | 149 ～150 | 1.40 (9H, s), 7.2～7.4 (6H, m), 7.54 (2H, d, J=9Hz), 11.24 (2H, br, s) |
| 2-Cl | H | 98 ～100 | 1.40 (9H, s), 7.27 (4H, d, J=9Hz), 7.3～7.7 (2H, m), 7.54 (2H, d, J=9Hz) |
| 2,5-diCl | H | 198 ～199 | 1.41 (9H, s), 7.08 (1H, dd, J=3Hz, 9Hz), 7.2～7.5 (2H, m), 7.26 (2H, d, J=9Hz), 7.66 (2H, d, J=9Hz), 11.0 (2H, br) |

Table 3 (5)

| $R^1$, $R^2$ | $R^3$ | m.p. (℃) | n. m. r. (δ value) ($CDCl_3$) |
|---|---|---|---|
| H | 2'-Cl | 184 ~186 | 1.40 (9H, s), 6.68 (1H, d, J=9Hz), 7.50 (1H, s), 7.2~7.7 (6H, m), 11.1 (2H, br) |
| 4-$NH_2$ | H | 89 ~91 | 1.44 (9H, s), 7.3~7.6 (8H, m) |
| 4-$NO_2$ | H | 122 ~125 | 1.41 (9H, s), 7.26 (2H, d, J=9Hz), 7.48 (2H, d, J=9Hz), 7.68 (2H, d, J=9Hz), 8.11 (2H, d, J=9Hz), 11.4 (2H, br) |
| 4-$CH_3CONH$ | H | 248 ~249 | 1.42 (9H, s), 2.16 (3H, s), 7.18 (2H, d, J=7Hz), 7.54 (2H, d, J=7Hz), 7.98 (2H, d, J=7Hz), 8.08 (2H, d, J=7Hz) |
| 4-$CF_3O$ | H | 120 ~123 | 1.42 (9H, s), 7.1~7.6 (8H, m) |

Table 3 (4)

| $R^1$, $R^2$ | $R^3$ | m.p. (℃) | n.m.r. (δ value) ($CDCl_3$) |
|---|---|---|---|
| 4-$CH_3CO$ | H | 168~169 | 1.41 (9H, s), 7.25 (2H, d, J=9Hz), 7.46 (2H, d, J=9Hz), 7.65 (2H, d, J=9Hz), 8.08 (2H, d, J=9Hz), 11.3 (2H, br) |
| H | 3'-$CH_3O$ | 74~77 | 1.32 (9H, s), 3.70 (3H, s), 6.64 (1H, d, J=7Hz), 6.70 (1H, s), 7.2~7.4 (5H, m), 7.50 (1H, d, J=7Hz), 10.7 (2H, br) |
| H | 2'-$CF_3$ | 92~94 | 1.36 (9H, s), 6.90 (1H, d, J=7Hz), 7.3~7.6 (6H, m), 7.8~7.9 (1H, m), 11.2 (2H, br) |

(Example 19) 4-(t-butylamino)diphenylsulfoxide hydrochloride

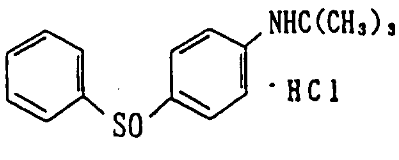

1.00 g (3.4 mmol) of 4-(t-butylamino)diphenylsulfide hydrochloride was dissolved in 10 ml methylene chloride, and 0.59 g (3.4 mmol) of m-chloroperbenzoic acid was added thereto for 10 min. at room temperature under a nitrogen stream, and the mixture was allowed to react for further 2 hours. Thereafter, the reaction solution was washed with 10 ml water, and the organic layer was concentrated under reduced pressure. The concentrate was subjected to silica gel column chromatography with methylene chloride/methanol = 50/1 (v/v) as an eluent to give 0.19 g of 4-(t-butylamino)diphenylsulfoxide (20 % yield). The resulting 4-(t-butylamino)diphenylsulfoxide was converted into the corresponding hydrochloride in the same manner as in Example 1 (92 % yield from the free form). The physical properties of 4-(t-butylamino)-diphenylsulfoxid e and the hydrochloride thereof are shown below.
4-(t-butylamino)diphenylsulfoxide
melting point: oil.
n.m.r. (δ value, $CDCl_3$): 1.38 (9H, s), 2.8-3.3 (1H, br), 6.69 (2H, d, J = 9 Hz), 7.3-7.5 (5H, m), 7.63 (2H, d, J = 9 Hz).
4-(t-butylamino)diphenylsulfoxide hydrochloride
melting point: 134-136 °C.
n.m.r. (δ value, $CDCl_3$): 1.34 (9H, s), 7.57 (2H, d, J = 9 Hz), 7.1-7.7 (5H, m), 9.38 (2H, d, J = 9 Hz), 11.2 (2H, br).

(Example 20) 4-(t-butylamino)diphenylsulfone hydrochloride

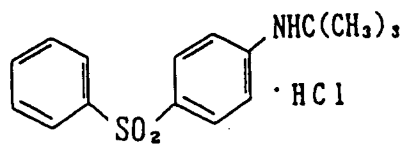

The same procedure as in Example 19 was followed except that the amount of m-chloroperbenzoic acid used was 1.77 g in place of 0.59 g. As a result, 0.43 g of 4-(t-butylamino)diphenylsulfone was obtained (38 % yield), and 0.45 g of the hydrochloride thereof was obtained (88 % yield from the free form). The physical properties of 4-(t-butylamino) diphenylsulfone and the hydrochloride thereof are shown below.
4-(t-butylamino)diphenylsulfone
melting point: oil.
n.m.r. (δ value, $CDCl_3$): 1.36 (9H, s), 4.3 (1H, br), 6.64 (2H, d, J = 9 Hz), 7.4-7.6 (3H, m), 7.70 (2H, d, J = 9 Hz), 7.8-8.0 (2H, m).
4-(t-butylamino)diphenylsulfone hydrochloride
melting point: 135-138 °C.

(Example 21) Tablet

Tablets of 9 mm diameter (200 mg per tablet) were manufactured in a usual manner from 100 g of Compound 1, 40 g of crystalline cellulose, 30 g of potato starch, 28 g of carboxymethylcellulose calcium and 2 g of magnesium stearate.

(Example 22) Capsule

Capsules with a content of 180 mg per capsule were manufactured in a usual manner from 120 g of Compound 2, 18 g of potato starch, 40 g of lactose and 2 g of magnesium stearate.

(Example 23) Injection

2 g of Compound 3 was dissolved in 1000 ml of physiological saline solution, injections were prepared by charging each ample with 2 ml of the solution, followed by sterilization thereof.

(Test Example 1) Action on the lipid level in blood

Male Wistar strain rats with weights of about 200 g were given a high cholesterol feed containing 1 % cholesterol and 0.5 % cholic acid. Simultaneously, a suspension of a test compound in 5 % aq. gum arabic was orally administered in an amount of 25 mg/kg/day. After the final administration, the animals were starved for 24 hours and then evaluated for serum total cholesterol (TC), high density lipoprotein-cholesterol (HDL-Ch) and serum triglyceride (TG) content. The control group was orally given 5 % aq. solution of gum arabic.

Table 4 shows TC decrease rate, HDL-Ch increase rate and TG decrease rate (%) relative to the control. In Table 4, test compounds Nos. 1 to 11 correspond respectively to the hydrochlorides of the compounds of Examples 1 to 11.

Table 4

| test compound | TC decrease rate | HDL-Ch increase rate | TG decrease rate |
|---|---|---|---|
| 1 | 95.7 | 39.4 | 48.5 |
| 2 | 43.4 | 18.8 | 19.5 |
| 3 | 33.4 | 15.2 | 81.2 |
| 4 | 31.1 | 21.3 | 25.1 |
| 5 | 38.0 | 26.5 | 40.0 |
| 6 | 71.4 | 31.8 | 59.0 |
| 7 | 45.1 | 25.6 | 34.8 |
| 8 | 39.9 | 22.2 | 10.2 |
| 9 | 57.0 | 32.3 | 12.5 |
| 10 | 57.9 | 32.3 | 10.0 |
| 11 | 73.1 | 36.6 | 26.0 |
| clofibrate | 1.2 | -1.6 | -0.9 |
| compound of Reference Example 3 | 3.6 | -0.7 | -2.1 |
| compound of Reference Example 4 | -16.2 | - | 30.0 |

(Test Example 2) Action on incorporation of LDL

hepatic cells were separated from the liver of a male Wistar strain rat weighing about 200 g, and the hepatic parenchymal cells were incubated in primary culture. 48 hours thereafter, a solution of a test compound in dimethylsulfoxide (DMSO) (10 μg/ml) was added thereto and incubated for 24 hours. Then, a radioisotope-labeled low density lipoprotein (LDL) was added thereto, and incubation was continued for further 4 hours. The cells were washed with physiological saline and then lysed with sodium dodecyl sulfate (SDS), and their radioactivity was determined in a liquid scintillation counter. The control group was treated in the same manner as above except for the addition of DMSO only.

Table 5 shows the ratio of incorporation of LDL (%) relative to the control. In Table 5, the test compounds 1 to 11 correspond respectively to the hydrochlorides of the compounds of Examples 1 to 11.

Table 5

| test compound | incorporation ratio of LDL |
|---|---|
| 1 | 65.3 |
| 2 | 33.4 |
| 3 | 23.3 |
| 4 | 23.2 |
| 5 | 17.3 |
| 6 | 43.2 |
| 7 | 30.2 |
| 8 | 25.6 |
| 9 | 40.1 |
| 10 | 35.7 |
| 11 | 45.2 |
| clofibrate | -10.5 |
| compound of Reference Example 3 | -15.7 |

(Test Example 3) Toxicity Test

Acute toxicity was examined in the Litchfield and Wilcoxon's method where 10 ICR male mice were orally given a test compound.

The animals given Compounds 1 to 12 in a dosage of 5000 mg/kg did not die, indicating that $LD_{50}$ was higher than 5000 mg/kg. The $LD_{50}$ of clofibrate was 1600 mg/kg.

INDUSTRIAL APPLICABILITY

As compared with clofibrate and 4-(N-t-butyl)methanesulfonylaminodiphenyl ether, the compound of the invention has a remarkable effect of depressing the lipid level in hypercholesteremia rats and is extremely low toxicity. Hence, the compound of the present invention is useful as lipid level depressant.

**Claims**

**1.** An N-t-butylaniline analog represented by formula (I):

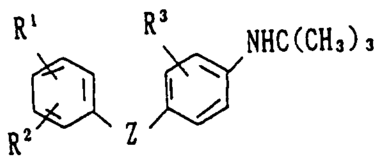

(I)

wherein $R^1$ and $R^2$ are the same or different and represent a hydrogen atom, halogen atom, amino group, nitro group, acetamido group, lower alkyl group, lower alkoxy group, halogeno lower alkyl group, halogeno lower alkoxy group or lower alkanoyl group, $R^3$ represents a hydrogen atom, halogen atom, lower alkoxy group or halogeno lower alkyl group, and Z represents S, SO or $SO_2$, or a salt thereof.

**2.** A compound according to claim 1, wherein in formula (I) Z is S.

**3.** A compound according to claim 2, which is 4-(t-butylamino) diphenylsulfide or a salt thereof.

**4.** A compound according to claim 2, which is N-t-butyl-4-(4-isopropylphenylthio)aniline or a salt thereof.

**5.** A compound according to claim 2, which is N-t-butyl-4-(4-tolylthio) aniline or a salt thereof.

**6.** A compound according to claim 2, which is N-t-butyl-4-(4-methoxyphenylthio)aniline or a salt thereof.

**7.** A compound according to claim 2, which is N-t-butyl-4-(4-bromophenylthio)aniline or a salt thereof.

**8.** A compound according to claim 2, which is N-t-butyl-4-(3-methoxyphenylthio)aniline or a salt thereof.

**9.** A compound according to claim 2, which is N-t-butyl-4-(3-trifluoromethylphenylthio)aniline or a salt thereof.

**10.** A compound according to claim 2, which is N-t-butyl-4-(3-chlorophenylthio)aniline or a salt thereof.

**11.** A compound according to claim 2, which is N-t-butyl-4-(2-chlorophenylthio)aniline or a salt thereof.

**12.** A compound according to claim 2, which is N-t-butyl-4-(2,5-dichlorophenylthio)aniline or a salt thereof.

**13.** A compound according to claim 2, which is N-t-butyl-3-chloro-4-(phenylthio)aniline or a salt thereof.

**14.** A compound according to claim 1, wherein in formula (I) Z is SO.

**15.** A compound according to claim 14, which is 4-(t-butylamino) diphenylsulfoxide or a salt thereof.

**16.** A compound according to claim 1, wherein in formula (I) Z is $SO_2$.

**17.** A compound according to claim 16, which is 4-(t-butylamino) diphenylsulfone or a salt thereof.

**18.** A lipid level depressant containing N-t-butylaniline analog defined in any one of claims 1 to 17 or a salt thereof as active ingredient.

## INTERNATIONAL SEARCH REPORT

International application No.
PCT/JP93/01516

**A. CLASSIFICATION OF SUBJECT MATTER**

Int. $Cl^5$ C07C317/36, 317/40, 323/37, 323/41, A61K31/135, 31/14, 31/16

According to International Patent Classification (IPC) or to both national classification and IPC

**B. FIELDS SEARCHED**

Minimum documentation searched (classification system followed by classification symbols)

Int. $Cl^5$ C07C317/36, 317/40, 323/37, 323/41, A61K31/135, 31/14, 31/16

Documentation searched other than minimum documentation to the extent that such documents are included in the fields searched

Electronic data base consulted during the international search (name of data base and, where practicable, search terms used)

CAS ONLINE

**C. DOCUMENTS CONSIDERED TO BE RELEVANT**

| Category* | Citation of document, with indication, where appropriate, of the relevant passages | Relevant to claim No. |
|---|---|---|
| A | JP, A, 60-255760 (Dr. Karl Thomae GmbH), December 17, 1985 (17. 12. 85), Claim; page 1 and upper right column, page 25 & DE, A1, 3419009 & EP, A1, 165422 & US, A, 4829058 | 1-17, 18 |
| A | JP, A, 61-64 (Richter Gedeon Vegyeszeti Gyar Rt), January 6, 1986 (06. 01. 86), Claims 4 and 6 &GB, A, 2155014 & FR, A1, 2560193 & DE, A1, 3506980 & US, A, 4710323 | 1-17, 18 |
| A | JP, A, 62-126155 (Mitsui Petrochemical Industries, Ltd. and another), June 8, 1987 (08. 06. 87), Claim, (Family: none) | 1-17 |
| A | GB, A, 1294136 (OCE-VAN DER GRINTEN N.V.), October 25, 1972 (25. 10. 72), Claims 1 and 2 & DE, A1, 2005462 & FR, A1, 2032329 | 1-17 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| January 5, 1994 (05. 01. 94) | January 25, 1994 (25. 01. 94) |
| Name and mailing address of the ISA/ Japanese Patent Office | Authorized officer |
| Facsimile No. | Telephone No. |